# EUROPEAN PATENT SPECIFICATION

(11) **EP 0 398 443 B1**
(45) Date of publication and mention of the grant of the patent: **17.11.1994**
(21) Application number: 90201244.2
(22) Date of filing: 16.05.1990
(51) Int. Cl.: C07K 7/10, A61K 39/00, G01N 33/569

(54) **Immunogenic compounds and their use in the preparation of genetically unrestricted synthetic vaccines and in the immunoenzymatic determination of antisporozoite antibodies of plasmodium malariae**
Immunogen-Verbindungen und deren Benutzung in der Herstellung von genetisch unbeschränkten synthetischen Impfstoffen und bei der immunoenzymatischer Bestimmung von antisporozoiden Antikörpern von Plasmodium malariae
Composés immunogènes et leur emploi dans la préparation des vaccins synthétiques génétiquement illimités et dans la détermination immunoenzymatique d'anticorps d'antisporozoite de plasmodium malariae

(30) Priority: 19.05.1989 IT 2055389; 23.03.1990 IT 1980090
(43) Date of publication of application: 22.11.1990
(73) Proprietor: ENIRICERCHE S.p.A., 20121 Milano (IT)
(72) Inventor: Pessi, Antonello, I-00199 Rome (IT); Bonelli, Fabio, I-00100 Rome (IT); Chiappinelli, Lorella, I-00198 Rome (IT); Bianchi, Elisabetta, I-00195 Rome (IT); Del Giudice, Giuseppe, CH-Hermance, Geneva (CH)
(74) Representative: Fusina, Gerolamo

(56) References cited:
- GB-A- 2 199 038
- GB-A- 2 199 140
- CHEMICAL ABSTRACTS, vol. 98, 1983, Columbus, OH (US); R.M. KAMP et al., p. 350, no. 175739y#

## Description

This invention relates to a new class of immunogenic compounds (I), their synthesis process and their use as diagnostics antigens in the determination by immunoenzymatic assay of antisporozoite antibodies of Plasmodium malariae and in the preparation of a genetically unrestricted Plasmodium malariae antisporozoite vaccine.

The invention also relates to the use of compounds of formula (I) as carrier molecules to improve the immunogenicity of haptens. The term "hapten" means a molecule able to bind specific antibodies (antigenic) but not to induce their formation or to induce it only at a low antibody level (non-immunogenic).

In particular, haptens include polypeptide or polysaccharide molecules (thymus-independent antigens) isolated and purified from pathogenic agents or short peptides with a sequence corresponding to one or more B epitopes of a natural antigen obtained by chemical synthesis or via recombinant DNA.

The term antigen means a molecule able to induce the formation of specific antibodies (immunogenic) and to react therewith (antigenic).

Natural microorganisms, cells and their soluble products are antigens.

The etiological agent of malaria is a protozoon of the Plasmodium genus which is transmitted to the vertebrate host (for example man) by the sting of the Anopheles mosquito. Of the four species which are infective to man, namely Plasmodium malariae (P. malariae), Plasmodium ovale (P. ovale), Plasmodium falciparum (P. falciparum) and Plasmodium vivax (P.vivax), these latter two cause most of the morbidity and mortality associated with the disease.

Malaria is currently one of the major health problems at world level. In fact, every year this disease affects about 250 million people to result in initial infancy in a mortality which can attain 50% of the cases.

This explains the requirement to develop an antimalaria vaccine able to give man a protective immunity against the pathogenic agent which causes the infection.

Malaria parasites develop by a multistage cycle in which the host is presented with a very large number of antigenic components, each development form of the parasite containing different stage-specific antigens.

In an attempt to identify protective plasmodial antigens, the interest of researchers has been directed towards those exposed to the immune system anti present both on the parasite surface and on the membrane of the infected red corpuscle.

Particular interest has been shown in the study of Plasmodium sporozoites in that the preparation of a vaccina against this stage, if completely effective in man, is able to prevent plasmodium development in the host and thus induce a sterile protective immunity.

The administration of sporozoites irradiated with X-rays to rodents, monkeys and man confers a protective immunity which is stage-specific but not strain-specific [Cochrane A.H. et al., (1980) Academic Press, New York pp 163-202).

In this respect, on incubating Plasmodium sporozoites with antisera (antibodies) obtained from animals immunized with X-ray irradiated sporozoites of the same species, a precipitate forms on the surface indicating that said antibodies recognize the specific antigens.

The use of monoclonal antibodies has made it possible to identify those antigens which pertain to a family of polypeptides (Circumsporozoitic protein or CSP) which cover the entire surface of the sporozoite and induce a specific antibody response which provides protection against malarial infections.

Recent developments in the molecular biology field have made it possible to identify the coding genes for Plasmodium antigenic proteins.

In particular, the coding genes for P.falciparum, P.vivax and P.malariae CSP have been cloned and sequentiated.

An analysis of the characteristics of said proteins has shown the presence of a central immunodominant domain or B epitope formed of units which repeat a certain number of times.

For P.falciparum said immunodominant epitope consists of the tetrapeptide Asn-Ala-Asn-Pro (NANP) repeated 37 times and 4 quadruplets with the amino acid sequence Asn-Val-Asp-Pro (NVDP). whereas for P.vivax and P.malariae it consists of Asp-Arg-Ala-Asp/Ala-Gly-Gln-Pro-Ala-Gly (DRAD/AGQPAG) and Asn-Ala-Ala-Gly (NAAG) respectively.

In certain experimental models, anti-repeating unit antibodies were able to provide protective immunity against experimental infection [Potocnjak P. et al., (1980), J. Exp. Med., 151:1504; Gysin J. et al., (1984), J. Exp. Med., 160:935; Zavolla et al., (1987), J. Exp. Med., 167:1591). Consequently polypeptides comprising the B epitopes of the CSP proteins have been considered valid candidates in the development of Plasmodium antisporozoite vaccines.

However, studies effected in various laboratories have shown that the ability of mice to respond to synthetic or recombinant peptides of the repetitive sequence of the CSP of P.falciparum [Good M.F. et al., (1986), J. Exp. Med., 164:655; Del Giudice G. et al., (1986), J. Immunol., 137:2952; of P.vivax [Nardin E.H. et al. (1988), Eur. J. immunol., 18:1119], of P.yoelli and P.berghei [Hoffman S.L. et al., (1989), J. Immunol., 142:3581] is genetically controlled by the H-2 genes of the murine histocompatibility complex. This means that only mice with haplotype H-2^{b} or H-2^{K} (responder mice) are able to produce specific antibodies.

For these reasons said synthetic peptides, even if genetically restricted in man, are not suitable for the development of antimalaria vaccines.

Immune response to an agent foreign to an organism is known to imply the cooperation of different types of cells, namely antigen presenting cells or APC, B lymphocytes (antibody producers and able to function as APC ), T-helper or Th lymphocytes, and T-cytotoxic lymphocytes responsible for the direct killing of cells infected with the pathogen.

The immunity can be of humoral type (mediated by the antibodies produced by the B cells) or of cell type.

Considering humoral immunity, the minimum requirement for initiation of an effective immune response is the recognition by the B lymphocytes circulating within the organism of an antigenic determinant of the foreign substance (B epitope) and recognition by the Th cells of a determinant, generally different than the B, of the substance itself (T epitope).

Two peptide sequences must therefore be present in the synthetic vaccine as the minimum requirement for its effectiveness, namely the B epitope and the T epitope.

While identification of the B epitope or epitopes contained in a natural antigen is facilitated by the ability to use natural anti-pathogenic antibodies as reagents, location of the T epitopes presents very complicated problems.

This is because the first case involves direct interaction between the anti-pathogenic antibody and the antigen whereas in the second case the activation of the T specific cell clone for an epitope of the antigen occurs only after this has been interiorized by an APC, processed by proteolysis or denaturation or both into short peptide fragments, and then re-exteriorized on the surface of the APC in association with a membrane molecule of the class II cell (for Ths) coded by a gene of the major histocompatibility complex (MHC). The Th lymphocyte receptor therefore recognizes not the free antigen but the complex formed from its fragment and a part of the MHC class II molecule.

These molecules are grouped into three families DR-DP and DQ for man (HLA) and IA and IE for the mouse and are extremely polymorphous.

The polymorphism is due to a large number of alleles or each of the coding genes for this molecule.

This diversity allows a large number of combinations in one chromosome (haplotype).

It is therefore very difficult to find unrelated individuals having an identical MHC.

Precisely because of the fact that only some of the peptides deriving from the degradation of the antigen are able to associate with a histocompatibility antigen of the host, the host gene set which is coding for said antigens in fact restricts the number of peptides able to form a complex with them, and which is an essential requirement for activation of the Th lymphocytes.

The term "genetic restriction" of the T epitopes is therefore used in the sense that said peptide fragments are restricted in their interaction with the T lymphocyte receptor by the proteins with which they are associated. This is the reason for the difficulty in identifying within a natural antigen the T epitope or epitopes with no or minimum genetic restriction suitable for developing synthetic acellular vaccines with wide effectiveness, ie able to induce protective immunization against the pathogenic agent of interest in individuals with different MHC gene sets.

Up to the present time this difficulty has been very often overcome by using as the T epitope source a macromolecule, also known as a carrier, to which a natural or synthetic peptide or polysaccharide hapten deriving from a pathogenic agent of interest is covalently linked.

Examples of carriers suitable for this purpose are the tetanus toxoid (TT), the diphtheria toxoid (CRM), serum albumin and lamprey hemocyanin (KLH), these giving the resultant conjugate minimum genetic restriction.

Conjugates comprising said carriers, also known as universal carriers, are able to function as T cell clone activators in individuals having very different gene sets.

In this respect, most of the animals used for in vivo tests and having different gene sets are able to activate an antibody response towards the conjugate antigen, ie they are responders. Even though this approach to the problem has proved effective, the use of macromolecular hapten carrier conjugates in an immunization process against a pathogenic agent still has numerous drawbacks due to the difficulty of standardizing the various preparation stages, the possible alteration of the antigenic properties of the hapten as a result of the conjugation reaction [J.P. Briand et al., J. Immunol. Methods, 78 (1985) 59-69], and finally the phenomenon known as "epitope suppression induced by the carrier" which causes suppression of anti-hapten antibody production in an individual already immunized with the carrier alone (H. Etlinger et al., J. Immunol., 140 (1988), 626). This phenomenon is observed whenever the carrier used is a protein to which the host has already been exposed, such as the tetanus toxoid used for anti-tetanus vaccination. Moreover, the use of short peptide sequences as immunogens, even comprising both a B epitope and a T epitope of the natural antigen, has generally led to a much more genetically restricted immune response, ie, a lesser number of responder animals and/or individuals (A. R. Togna et al., J. Immunol. 137 (1986) 2956-2960; Del Giudice et al., J. Immunol. 137 (1986) 2962-2955 and Del Giudice et al., Immunology 63 (1988) 187-191).

An important approach to the direct synthesis of a peptide-antigen matrix by the solid phase-method, called the multiple antigen peptide system (MAP), is disclosed in J. P.Tam (1988) "Synthetic peptide vaccine design: Synthesis and Properties of a high-density multiple antigenic peptide system", Proc. Natl. Acad. Sci. USA, 85:5409-5413.

In addition to the problem of developing a highly-protective antimalarial vaccine, there is also that of accurately defining the epidemiologic situation in endemic areas: mass vaccination programs are not feasible, so that the initial step is the vaccination of the group which are mostly at risk. Proper malaria control requires the knowledge of the zones where the transmission of the infection is very high, that which involves a large-scale continuous capillary monitoring.

This requires diagnostic methods having no intricate techniques or apparatus, which are generally little available in tropical zones, while making it possible to take samples in areas accessible with difficulty, without having the samples stored at a low temperature and/or immediately transported to the monitoring centre. Immunoenzymatic assays (EIA) such as the ELISA (enzyme linked immunosorbent assay) are particularly suitable for epidemiologic investigations as they enable a large number of samples to be tested simultaneously, are simple to carry out and do not require sophisticated equipment. Setting up such tests requires the availability of a specific antigen of high purity producible in adequate quantity and under completely reproducible conditions. Peptides corresponding to the immunodominant sequence of an antigen such as the CS protein and prepared by chemical synthesis are particularly suitable for the purpose.

The characteristics which such peptides must possess from the immunodiagnostic activity viewpoint are:
a) specificity, ie they must be able to effectively discriminate between the antibody molecules present in the examined samples and choose only those which react towards the desired antigen;
b) ability to coat a solid support such as an ELISA plate and to withstand both washing and the applied treatment until the final colour development reaction.

Recently, Lal A.A et al., [(1988), J. Biol., 263:5495-5498] have described the synthesis of the peptide (NAAG)₅ and its use in an immunofluorescence assay (IFA) for a study on the relationship between antibodies which recognize the synthetic peptide and those which recognize P.malariae sporozoites.

However this peptide, as demonstrated in the following Example 2 which describes the use of the peptide (NAAG)₆-NA, is not suitable as an antigen for the development of an immunoenzymatic assay such as the ELISA because of its poor ability to adhere to a support such as the wells of microtitration plates, which are generally of polystyrene construction.

In this respect a high concentration (3-5 µg/ml) is required to obtain a coating of this peptide.

When operating under these conditions, although demonstrating a certain capacity to detect antisporozoite monoclonal antibodies (Mab) of P.malariae the assay lacks sensitivity and has too low a detection threshold to be a reliable epidemiologic instrument. The tendency of peptides to be absorbed non-covalently on a solid support varies from sequence to sequence. In particular, in the case of sequences without an abundance of hydrophobic sequences and/or residual charges it has been seen that the ability to bind to a substrate is related to its molecular weight. In some cases it is known to improve the adhesiveness of the peptide by conjugating it with a protein of good binding ability such as bovine serum albumen (BSA).

However, tests conducted using anti-CSP monoclonal antibodies of P.malariae have shown that the (NAAG)₆-NA-BSA conjugate although being an improvement over (NAAG)₆-NA alone still does not represent the optimum solution to the problem.

This is probably due to the insufficient level of conjugation obtained in the conjugate used in the assay, ie a low ratio of number of (NAAG)₆-NA molecules to the number of BSA molecules. In any event, conjugates of this type still have drawbacks deriving from the fact that the antigen peptide chains, ie the (NAAG)₆-NA, on the surface of the BSA are much spaced apart and do not allow the cooperative bonding of the antisporozoite antibodies of P.malariae. Such a bond in fact occurs when the antigen binds simultaneously to the two binding sites present in the antibody molecule, resulting in an interaction of higher affinity and consequently a greater assay sensitivity.

Finally, from an industrial viewpoint, it is difficult to ensure standardization of preparation procedures and consequently the reproducibility of the conjugate obtained.

Again, the peptide (NAAG)₆-NA is unsuitable for the development of a P.malariae anti-CSP vaccine as it is poorly immunogenic and genetically restricted.

It has now been found possible to overcome the problems of the known art by a new class of compounds having the desired immunogenic properties.

Consequently one aspect of the present invention is a new class of immunogenic compounds consisting of a core of n lysine residues of α and ε amide linkage bound to a number m of repetitions of the repetitive sequence of the P.malariae CSP.

A further aspect of the present invention is the use of said compounds for determining antisporozoite antibodies of P.malariae in a biological sample by means of an immunoenzymatic assay.

A further aspect of the present invention is a diagnostic kit for detecting antisporozoite antibodies of P.malariae by an immunoenzymatic assay comprising a solid support on which an immunologically effective quantity of a compound as heretofore defined is adsorbed. A further aspect of the present invention is an immunologically active composition suitable as a genetically unrestricted P.malariae antisporozoite vaccine containing an immunologically effective amount of a compound as defined hereinafter. A further aspect is the use of the compounds in question as hapten carriers for preparing immunologically active vaccine compositions to be used as genetically unrestricted anti-hapten vaccines.

The invention, according to one of its aspects, provides: An immunogenic compound having the general formula: 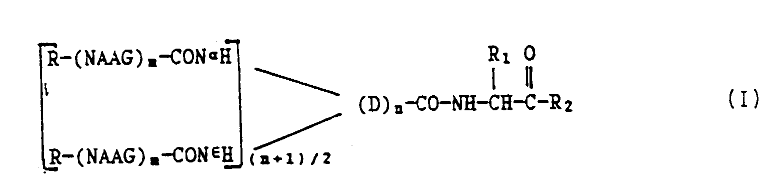
wherein:
n is an odd integer from 1 to 15
m is an integer from 3 to 40
N is L-Asp;
A is L-Ala;
G is L-Gly;
D is L-lysine, or a branched poly-(L-lysine) having a number, n, of L-lysine-amino-acid residues of an α and ε amide linkage;
R₁ is the side-chain of an amino-acid residue selected from L-Asp, L-His, L-Cys, L-Gln, L-Thr, L-Ala, L-Leu, L-Met, L-Phe, L-Glu, L-Arg, L-Tyr, L-Asn, L-Ser, L-Gly, L-Val, L-Ileu, L-Pro, and L-Trp;
R₂ is either -OH or -NH₂,
R is either hydrogen or an acyl,
and the pharmaceutically acceptable acid-addition or basic-addition salts thereof. The single-letter code adopted for the aminoacids is: Asp (D), Lys (K), His (H), Cys (C), Gln (Q), Thr (T), Ala (A), Leu (L), Met (M), Phe (F), Glu (E), Arg (R), Tyr (Y), Asn (N), Ser (S), Gly (G), Val (V), Ileu (I), Pro (P), Trp (W).

For the purposes of the present invention the term "acyl radical" identifies those acyl radicals deriving from linear or branched chain C₁-C₆ alkanoic acids such as formyl, acetyl, propionyl, succinoyl and the aromatic acyl radicals deriving from benzoic acid and substituted benzoic acid such as benzoyl, 4-nitro-benzoyl and 2,3,4-trimethoxybenzoyl.

As used herein the term "pharmaceutically acceptable salts" indicates those salts of acid or basic addition in which the anion or respectively the cation are non-toxic and innocuous when the compounds are administered as salts of addition at therapeutically effective doses.

Acids able to form pharmaceutically acceptable salts of acid addition with the compounds of formula (I) can be chosen from inorganic acids such as hydrochloric acid, hydrobromic acid, phosphoric acid or sulphuric acid, and carboxylic organic acids such as formic acid, acetic acid, propionic acid, lactic acid, malonic acid, succinic acid and the sulphonic organic acids such as methansulphonic acid or naphthalenesulphonic acid.

Bases able to form pharmaceutically acceptable salts with the compounds of formula (I) comprise for example the mineral bases such as sodium or potassium hydroxide, ammonium hydroxide, and the organic bases such as triethylamine, triethanolamine etc.

These salts of addition can be prepared either directly during the synthesis of the compounds of formula (I) or can be obtained by conventional methods by reacting said compounds (I) with one or more equivalents of the chosen acid or base.

A preferred group of compounds of formula (I) according to the present invention comprises those compounds in which:
n is an odd whole number between 3 and 15;
R₁, where possible, is the side chain of an amino acid residue other than N, A and G;
R is hydrogen or a metabolically labile acyl radical, or an acyl radical in which the bond with the amino group is quickly broken during the initial stages of the metabolic path of the product and which does not have toxic effects or contraindications in therapy, at the concentration in which the compound of formula (I) exhibits the desired effect.

According to the present invention, the compounds (I) are indicated hereinafter as MAP.[(-NAAG)ₘ]ₙ.

The compounds of formula (I) can be prepared by solid phase condensation using one of the known methods. Preferably the flow polyamide method is used as described by A. Drylan and R.C. Sheppard (J.Chem. Soc. Perkin Trans. I, 1986, 125-137) which compared with the classical Merrifield procedure [R.B. Merrifield (1963), J. Am. Chem. Soc., 85:2149-2154] has the following advantages:
- optimum solvation of the entire system as the matrix (of polyacrylamide type), the peptide (a functionalized polyamide) and the solvent used in all the operations (DMF) are of identical chemical nature;
- completion of the reaction after the initial coupling conducted at ambient temperature;
- possibility of on-line analytical monitoring of the reaction by following the spectrophotometric trace of the column eluate. The analysis, thus effected on the total column contents, avoids any potential risk due to non-homogeneous sampling. Analytical monitoring of the coupling reactions is a very important element in the synthesis of the compound according to the present invention in that synthetic compounds of such dimensions are difficult to purify on termination of the synthesis. Errors such as truncated peptides or peptides lacking an internal amino acid are amplified and are difficult to analyze and remove;
- finally, the possibility of cleaving the compound (I) from the resin, in the absence of particularly reactive side chain chemical groups such as Met, Cys, Trp, Arg or Tyr, with a trifluoroacetic acid (TFA)/water (90:10 v/v) mixture, by which on termination of the reaction the peptide can be recovered by simple lyophilization.

According to the present invention compounds of formula (I) are synthesized in the solid phase by a process comprising:
a) condensing the first amino acid residue protected at the α-amino group and possibly at the reactive side chain functions on an insoluble support by an esterification reaction between the activated terminal carboxylic group and the handle of the solid support;
b) removing the protecting group from the α-amino group of the amino acid residue esterified as in stage a);
c) condensing the L-lysine (K) amino acid residue protected at the α and ε amino groups by an acylation reaction between the deprotected amino group of the amino acid residue bound to the resin and the activated carboxyl group of K;
d) removing the protecting groups from the α and ε amino groups of the first lysine residue;
e) condensing the K residues required to obtain the required polylysine core by an acylation reaction between their activated terminal carboxyl and the deprotected α and ε amino groups of the lysine residue and/or residues bound to the resin;
f) removing the protecting groups from the α and ε amino groups of the lysine residues and condensing the next amino acid residues by an acylation reaction between the activated terminal carboxyl and the deprotected α and ε amino groups of the amino acid residue of the growing peptide chain until the compound of formula (I) is complete;
g) cleaving the obtained compound of formula (I) from the insoluble support by acid hydrolysis and recovering and purifying the compound (I) by dialysis and/or chromatography. The insoluble solid supports can be chosen from commercial polyacrylamide resins such as Pepsyn^{R} K functionalized with norleucine (Nle) as internal reference amino acid and with a peptide handle at the resin, such as 4-hydroxymethylphenoxyacetic acid

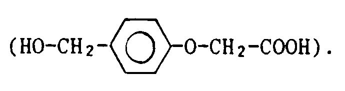

The functionalization reaction is conducted at ambient temperature (20-25°C) in the presence of an equivalent quantity of 1-hydroxybenzotriazole (HOBt).

### Stage a)

The first amino acid residue forms the point of connection to the resin on which the compound (I) is synthesized, an is chosen from the amino acid residues L-Asp, L-His, L-Cys, L-Gln, L-Thr, L-Ala, L-Leu, L-Met, L-Phe, L-Glu, L-Arg, L-Tyr, L-Asn, L-Ser, L-Gly, L-Val, L-Ileu, L-Pro and L-Trp. Preferably, this residue is not contained in NAAG nor in any epitope linked to its terminal carboxyl.

In stage a) of the process according to the present invention the amino acid residue is condensed at the resin handle after protecting the α-amino group and any reactive side chain functions, and activating the terminal carboxyl group by esterification.

Examples of protecting groups for the α-amino group are benzyloxycarbonyl, triphenylmethyl (trityl, Trt), tert.amyloxycarbonyl, 2-nitrophenyl sulphonyl, fluorenylmethyloxycarbonyl (Fmoc) and tert.butyloxycarbonyl (Boc).

The preferred of these protecting groups are Fmoc and Boc, which are removable under mild conditions. The protective Fmoc group is particularly preferred. Any reactive functional groups present in the amino acid side chains are protected using conventional protecting groups. Generally, protecting groups are used which are stable under the conditions under which the α-amino group protector is removed. Examples of such groups are tert-butyl or tert-amyl for the carboxyl and hydroxyl of tyrosine, the Boc, trifluoroacetyl, ortho-bromobenzyloxycarbonyl (o-Brz) or benzyloxycarbonyl group for the amino group of lysine, the trityl or N^{G}-4-methoxy2,3,6-trimethyl-benzenesulphonyl (Mtr) group for the guanidine group of arginine, the tert-butylester (OBu^{t}) group for aspartic and glutamic acid, and the triphenylmethyl or Boc group for histidine. These protecting groups can be removed simultaneously with the cleavage of the compound (I) from the resin.

According to the present invention the terminal carboxyl group of the amino acid residue, suitably protected at the α-amino group and at the side chain functional groups, is activated using equimolar quantities of the amino acid and a phenol derivative to form the active ester at the terminal carboxyl.

Phenol derivatives usable in the process of the present invention are fluorinated or chlorinated derivatives such as pentafluorophenol, trichlorophenol, pentachlorophenol and para-nitrophenol. The activated amino acid is then condensed (esterification) as ester at the resin handle.

Specifically, the esterification reaction is conducted in the polar organic solvent dimethylformamide (DMF) in the presence of 4-dimethylaminopyridine (DMAP) and N-methylmorpholine (NMM) at ambient temperature for a period of about 15 minutes.

### Stage b)

In stage b) of the process of the present invention the protecting group for the α-amino group of the amino acid residue is removed by conventional methods.

If the protecting group for the α-amino group is or example Fmoc, it is removed using a piperidine:dimethylformamide (20:80 v/v) mixture.

### Stage c, d) and e))

Stages c), d) and e) of the process of the present invention involve the condensation of the first amino acid residue, L-lysine, suitably protected at the α and ε amino groups and activated at the terminal carboxyl group by an acylation reaction at the deprotected α-amino group of the amino acid residue linked to the resin.

The terminal carboxyl group can be activated using one of the known methods. In a first embodiment of the present invention the activation reaction is effected using dicyclohexylcarbodiimide (DCC).

The acylation reaction can be conducted by conventional methods in an inert organic solvent possibly in the presence of catalysts. Inert organic solvents are chosen from chlorinated aliphatic hydrocarbons, aliphatic ketones or alkyl esters. N,N-dimethylformamide (DMF), methylene chloride, ethyl acetate or tetrahydrofuran are preferably used.

The catalysts are chosen from those generally used in peptide synthesis, 1-hydroxybenzotriazole being preferably used in a concentration of 1-2 equivalents.

The temperature at which the acylation reaction is conducted can vary from -10°C to 40°C, preferably 20-25°C, and the corresponding reaction time is that required to complete or substantially complete the reaction.

On termination of the acylation reaction the protecting groups are removed from the α and ε amino groups of the L-lysine residue. When compounds of formula (I) are synthesized in which D is a poly(L-lysine) with a number of amino acid residues exceeding 1, in the various coupling reactions increasing quantities of the L-lysine amino acid residue protected at the α and ε amino groups and activated at the terminal carboxyl group are used, because of the progressive increase of amino groups present on the resin. Typically, the protecting groups for the lysine α and ε amino groups are removed between one coupling reaction and the next operating as described for stage b), with repeated washing with DMF.

### Stage f)

After removing the protecting groups for the α and ε amino groups of the L-lysine amino acid residue and/or residues, the subsequent amino acids, suitably protected by protecting groups chosen from those of the known art and activated at the terminal carboxyl group as esters operating as described in stage a), are condensed by an acylation reaction at the deprotected α-amino group of the growing compound (I).

When the synthesis of the compound (I) is complete and the protecting group has been removed from the α-amino group, this can be acylated to prevent the compound having a polycation structure. For this purpose, C₁-C₆ linear or branched chain alkanoic acids can be used, such as formyl, acetyl, propionyl, succinoyl and the aromatic acyl radicals deriving from benzoic acid and substituted benzoic acid such as benzoyl, 4-nitro-benzoyl and 2,3,4-tri-methoxybenzoyl.

Preferably, metabolically labile groups, or an acyl radical in which the bond with the amino group is quickly broken during the initial stages of the metabolic path of the product and which does not have toxic effects or contraindications in therapy at the concentration at which the compound (I) exhibits its desired effect are used. The acetyl radical (Ac) is particularly preferred and is introduced via the (CH₃CO)₂O reactant.

### Stage g)

In stage g) of the process, the resin-peptide is removed from the column, repeatedly washed with an ancohol such as methanol or ethanol or mixtures thereof, and then dried in a vacuo. The compound (I) can then be cleaved from the resin by conventional methods. A trifluoroacetic acid (TFA)/water mixture (90/10 v/v) mixture is preferably used. On termination of the reaction, the expected compound (I), either as such or in the form of a salt of addition, is recovered and then purified by dialysis and/or by conventional chromatographic methods. The homogeneousness of the compounds is tested by tlc and HPLC, and their identity is determined by amino acid analysis and, where possible, by spectroscopic methods (NMR, mass spectrometry). According to another aspect, the invention provides an immunogenic compound comprising one or more haptens having the general formula: 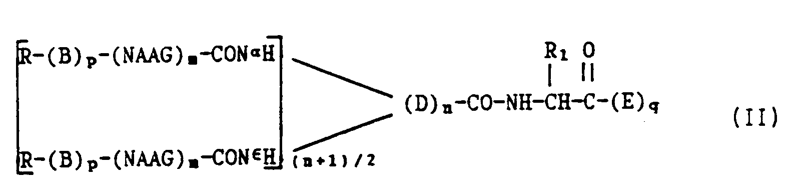
wherein:
n is an odd integer from 1 to 15;
m is an integer from 3 to 40;
N is L-Asp;
A is L-Ala;
G is L-Gly;
D is L-lysine, or a branched poly-(L-lysine) having a number, n, of L-lysine-amino-acid residues of an and amide linkage;
R₁ is the side-chain of an amino-acid residue selected from L-Asp, L-His, L-Cys, L-Gln, L-Thr, L-Ala, L-Leu, L-Met, L-Phe, L-Glu, L-Arg, L-Tyr, L-Asn, L-Ser, L-Gly, L-Val, L-Ileu, L-Pro, and L-Trp;
R₂ is either -OH or -NH₂,
R is either hydrogen or an acyl,
B and E, equal to, or different from one another, are haptens selected from the B-epitopes of the CSP of Plasmodium falciparum, (NANP)ᵣ, and of Plasmodium vivax, (DRAD/AGQPAG)ᵣ, wherein r is an integer from 3 to 40;
p and q are 0 or 1, with the proviso that they are never 0 simultaneously,
and the pharmaceutically acceptable acid-addition or basic-addition salts thereof.

Examples of haptens present in (II) are polypeptides or polysaccharides derived from diferent viral and bacterial pathogenic agents such as meningocci, pneumococci, Haemophilus influenzae, B.haemolytic streptococci, Escherichia coli, Bordetella pertussis, and Plasmodium. In particular, the haptens can be chosen from the synthetic peptides having the sequence corresponding to that of one or more B epitopes of a natural antigen. According to an embodiment, the hapten of (II) is chosen from the B epitopes of the CSP of P. falciparum, (NANP)ᵣ and of P.vivax, (DRAD/AGQPAG)ᵣ, wherein r is an integer from 3 to 40, the 5-16 range being preferred. The compounds (II) can be prepared by conjugation between a compound (I) in which R₂ is -OH and R₁ is H and the suitable hapten and/or haptens using the conventional methods such as homogeneous phase condensation in an inert organic solvent or using a cross-linking agent of the glutaraldehyde type, as reported for example by Avremas and Ternynck (Immunochemistry 6, 53 (1969)).

According to one embodiment of the present invention, compounds of formula (II) in which B is a polypeptide hapten and q is 0 can be prepared by conjugating the suitably protected amino acids by an acylation reaction at the (NAAG)ₘ until the hapten polypeptide sequence has been completed.

Compounds of formula (I) according to the present invention are antigens particularly suitable for the development of an immunodiagnostic assay of ELISA type for determining antisporozoite antibodies of P.malariae in samples such as whole blood, serum and blood spots, ie blood samples absorbed on filter paper and eluted at the moment of the test.

In this respect, said compounds are characterised by a small polylysine core and a high density of stratified antigen within it (most, 94%, of the peptide consisting of the antigen) which facilitates the cooperating binding.

This situation is in net contrast to that of conventional peptide-carrier conjugates in which a low antigen density is present distributed randomly on the BSA macromolecular support in an unidentified form.

In addition the synthesis process, which is highly controlled and reproducible, is well suited to industrial scaling-up.

In accordance with the present invention some of the compounds of formula (I) tested in mice having different gene sets were able to induce a genetically unrestricted high-level P.malariae antisporozoite antibody response.

Consequently, compounds of formula (I) and their pharmaceutically acceptable salts of acid or basic addition can be used for formulating compositions suitable as P.malariae antisporozoite vaccines able to provide genetically unrestricted protective immunity.

In addition, compounds of formula (II) in which the hapten is the B epitope of P.falciparum CSP, when tested in mice which were non-responders to (NANP)ₙ, were able to produce a high level of anti-NANP antibodies.

In conclusion, the compounds of formula (I) according to the present invention are suitable as universal carrier molecules in the preparation of immunogenic conjugates of formula (II) able to induce genetically unrestricted protective immunity or with a low genetic restriction against different pathogenic agents.

The vaccines are generally prepared by lyophilizing a suitable dose of a compound of formula (I) and reconstituting the vaccine at the moment of use with a suitable aqueous vehicle such as distilled water, physiological solution or suitable buffers.

This vaccine can also contain other components such as stabilizers, preservatives etc. In the case of oral formulations, the vaccine can contain flavourings as known in the pharmaceutical art, and be formulated in gastro-resistant dosage forms. In general the antigen doses used are chosen according to the method of administration, the patient's age and body weight, and the gravity of the disease.

The following experimental examples illustrate but do not limit the invention.

### Brief description of the figures

Figure 1 - This shows the antibody response to the MAP[(NAAG)₆]₈ peptide in strains of mice with different H-2 haplotypes. The mean optical densities at 414 nm with sera diluted 1:100 are shown ± the standard deviation.
Figure 2 - The horizontal axis represents the concentrations of the peptides MAP[(NAAG)₆]₈ (□), (NAAG)₆ (△) and Lys₇ (o) with which the sera withdrawn from mice immunized with MAP[(NAAG)₆]₈ before the ELISA. The vertical axis represents the OD values at 414 nm. Each point represents the average of two values.
Figure 3 - This shows the carrier effect of the peptide MAP[(NAAG)₆]₈ on the production of anti-(NANP)₄₀ antibodies. The symbols represent the average of two values.

The following examples illustrate the invention.

### EXAMPLE 1

### Synthesis of the compound MAP[(NAAG)₆]₈

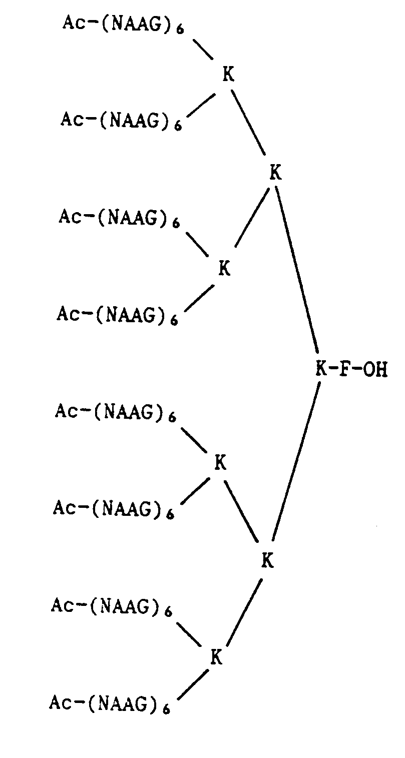

The reactions involving the functionalization of the resin, the esterification of the first amino acid residue (F) thereat and the acylation of the L-lysine residues are conducted batchwise whereas the subsequent synthesis operations are conducted using an automatic peptide synthesizer (Biolynx model 4170, Pharmacia-LKB) in accordance with the manufacturer's instructions and a glass column suitable for flow synthesis (Omnifit, 10 x 1 cm).

1 g of commercial Pepsyn^{R} K resin (Milligen, Millipore Co., Beldford, MA, USA) functionalized with 0.1 meq/g of sarcosine methylester is treated overnight at ambient temperature (20-22°C) with distilled ethylenediamine (20 ml).

The resin is then washed 10 times with dimethyformamide (DMF), functionalized with NLe as internal reference amino acid (reaction with 0.3 mmoles of Fmoc-NLe-OpfP, where opfP signifies pentafluorophenylester) in the presence of 0.33 mmoles of 1-hydroxybenzotriazole (HOBt) for 45 minutes at ambient temperature. After removing the protecting Fmoc group by treatment with 10 ml of a piperidine/DMF (20:80 v/v) solution for 10 minutes at ambient temperature, with 10 intermediate washes with DMF, the resin is functionalized with the peptide reversible handle by reacting it with 0.03 mmoles of 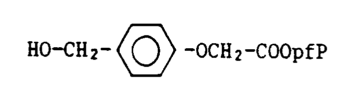
in the presence of 0.33 mmoles of HOBt for 45 minutes at ambient temperature.

The esterification of the first amino acid residue (F) is conducted under batchwise conditions at the handle.

Because of the high structural density of the compound, in order to reduce the initial functionalization of the resin a reaction time of 15 minutes less than normal (30 minutes) is used. In practice, 0.3 mmoles of Fmoc-(F)-OpfP, 0.03 mmoles of 4-dimethylaminopyridine (DMAP) and 0.3 mmoles of N-methylmorpholine (NMM) are dissolved in 3 ml of DMF and brought into contact with the resin for 15 minutes.

The resultant level of incorporation of amino acid F id 0.0107 mmoles/g of resin. As the amino acid derivative used for incorporating the L-lysine (K), ie Fmoc-K(Fmoc)OH, must be activated by reaction with dicyclohexylcarbodiimide (DCC), which involves precipitating dicyclohexylurea (DCU) which is only slightly soluble in the reaction medium, the three coupling reactions for the L-lysine residues are conducted batchwise using increasing quantities of Fmoc-K(Fmoc)OH because of the progressive increase in the amino groups present on the resin.

For the first coupling 0.075 mmoles of Fmoc-K(Fmoc)OH, 0.75 mmoles of DCC and 0.09 mmoles of HOBt are used, dissolved in 3 ml of DMF and brought into contact with the resin for 2.30 hours.

For the second coupling 0.15 mmoles of Fmoc-K(Fmoc)OH, 0.15 mmoles of DCC and 0.18 mmoles of HOBt are used under the above conditions.

Finally, for the third coupling 0.3 mmoles of Fmoc-K(Fmoc)OH, 0.3 mmoles of DCC and 0.36 mmoles of HOBt are used, again under the above conditions. The removal of the protecting Fmoc group between one coupling and the next and the intermediate washes are conducted as described heretofore with reference to the NLe coupling.

All the other operational cycles are conducted using an automatic peptide synthesizer (Biolynx model 4170, Pharmacia-LKB) in accordance with the manufacturer's instructions and a glass column suitable for flow synthesis (Omnifit, 10 x 1 cm).

Specifically, the wash cycle is conducted by passing DMF through the column at a rate of 3.5 ml/minute for times varying from 5 to 10 minutes, the Fmoc removal cycle consists of passing a solution of piperidine in DMF (20:80 v/v) through the column at a rate of 3.5 ml/minute for 10 minutes, and finally the acylation cycle (coupling) consists of dissolving the activated amino acid as pentafluorophenol ester (0.3 mmoles) together with 0.33 mmoles of HOBt in 3 ml of DMF and adding the resultant solution to the column (via the instrument pump at a rate of 2 ml/minute), then putting the system under recirculation for 45 minutes. All the reactions are complete on termination of the fixed periods, as determined by the spectrophotometric graph produced by the instrument plus colorimetric confirmation tests (E. Kaiser et al., Anal. Biochem., 1970, 34: 595), together with subsequent amino acid analysis of the final product. 24 complete acylation-removal cycles are conducted using the appropriate activated amino acids (Fmoc-G-OpfP, Fmoc-A-OpfP, Fmoc-N-OpfP) in sequence at a concentration of 0.3 mmoles per 3 ml of DMF in the presence of 1.1 equivalents (0.33 mmoles) of HOBt.

After removing the protecting group of the α-amino Asn residue (24th cycle), the amino group is acetylated by treatment with a solution of (CH₃CO)₂0 (0.3 mmoles) and NMM (0.3 mmoles) in 3 ml of DMF for a reaction time of 60 minutes.

The resin-peptide is removed from the column, transferred onto a glass filter of porosity 3 (G-3) and washed firstly with DMF, then with methanol and finally with ethyl ether and then dried under vacuum at ambient temperature for 18 hours. The peptide MAP[(NAAG)₆]₈ is cleaved from the resin by bringing 20 ml of a trifluoroacetic acid (TFA)/water (90:10 v/v) mixture into contact with 500 mg of dry resin at ambient temperature for 3 hours. On termination of the reaction the resin is filtered off, washed with 20 ml of the same mixture and then with 20 ml of water (cleaving yield 90%).

The pooled filtrates are evaporated under vacuum to eliminate the TFA, and the resultant acid solution is lyophilized (92 mg, 4.81 µmoles). An aliquot of the lyophilized product is used as such in the ELISA, and another aliquot is further purified by gel filtration in a 80 x 2.6 cm column of sephadex^{R} G-50, collecting the fractions corresponding to the main peak (flow = 36 ml/hour, eluent 0.1 M CH₃COOH, UV detection equal to 227 nm) and then used in an ELISA. The results obtained are identical for both the lyophilized products, thus indicating that the synthesis product can be used in the immunoenzymatic assay without any purification. The homogeneousness of the compound MAP[(NAAG)₆]₈ is confirmed by reverse-phase HPLC (a single narrow main peak is present accompanied by minor impurities). The purity estimated by integrating the UV signal is 85%.

HPLC conditions: column Vyrdac C-18, 15 x 0.4 cm, 300 Å, 5µm; eluent A = H₂O (0.1% TFA, 1% CH₃CN), eluent B = CH₃CN; linear gradient 10% B to 70% B in 15 minutes; flow 1.5 ml/minute. UV detection λ = 230 nm; product t_{R} = 6.02 minutes.

Analysis of the amino acid content of the MAP[(NAAG)₆]₈ gives:

| Phe | Lys | Asn | Ala | Gly |
|---|---|---|---|---|
| 1.00 | 7.10(7) | 44.85(48) | 94.08(96) | 51.47(48) |

the theoretical values being given in parentheses.

The overall synthesis yield, calculated on the first amino acid coupled to the resin, is 76%.

### EXAMPLE 2

### ELISA: Comparison between (NAAG)₆NA,(NAAG)₆NA-BSA and MAP[(NAAG)₆]₈

Nunc 96-well flat bottomed microtitration plates (Nunc-Immunoplate 1, Nunc, Roskilde, Denmark) are used. The plates are incubated overnight at ambient temperature (20-25°C) in a humid chamber with 100 µl/well of a solution of said peptides in phosphated buffer saline (PBS) of pH 7.2. After 4 washes with PBS containing 0.05% Tween 20 (PBS-T) the plates are saturated for 1 hour at ambient temperature with 200 µl of PBS-T containing 5% of powdered milk. 100 µl of human serum diluted in PBS-T containing 2.5% of powdered milk are then added doubly to each microplate well and incubated at ambient temperature for 1 hour. After 4 washes with PBS-T, 100 µl of peroxidase-antihuman rabbit IgG conjugate diluted 1:5000 in PBS-T containing 2.5% of powdered milk are added to each well. The plates are incubated at ambient temperature for 1 hour. After 4 washes with PBS-T, 100 µl of specific substrate (orthophenylenediamine of Fluka AG, Buschs, Switzerland: 0.4 mg/ml in 0.1 M citrate buffer pH 5.0 containing 0.01% of hydrogen peroxide) are added. The enzymatic reaction is blocked after 20 minutes by adding 50 µl of 2.5 N H₂SO₄. The optical density (O.D.) is read by absorbance at 492 nm using a Titertek Multiskan reader (Flow Laboratories, McLean, VA). For each analysis, plates not adsorbed with the antigen were also used, incubated with PBS at pH 7.2 and processed as the adsorbed plates. The results are expressed as the difference between the O.D.values obtained for the two plates. Use of the following optimizes the adsorption procedure:
a) Greisner, Dynatec and Titertek plates;
b) Na₂CO₃ pH 9.8 adsorption buffer (1 µg/ml);
c) concentrations of MAP[(NAAG)₆]₈ equal to 2, 1, 0,5 and 0,25 µg/ml.
d) dry treatment (washing plates with water and drying in a drier) and wet treatment (no drying of the plates after coating with BSA.

The antigen activity is measured using the monoclonal antibodies MAb POX anti P.malariae and sera originating from Africa and Italy.

The results obtained showed that:
- coating with carbonate buffer gives for all types of plates O.D. values which are higher by 15-20% than the values for PBS buffer (measured with MAb POX);
- for all plates the optimum antigen concentration is 1 µg/ml; a 2 µg/ml concentration gives O.D. values of about 5% higher and a 0.5 µg/ml concentration gives O.D. values of about 5% lower (measured using MAb POX);
- dry versus wet coating: the best results are obtained with dry coating. This lowers the X values of Italian sera by 25-49% and of African sera by 20-25%;
- Nunc plates give the best results in terms of specific/aspecific coupling ratio; on nunc plates the X value sera from the Italian samples is 29% of the X values for the African sera; on the other plates the mean of the Italian sera values reaches higher percentages, namely 31% on Greisner plates, 35% on Titertek plates and 36% on Dynatec plates.

The ELISA, conducted using the (NAAG)₆-NA at a concentration of 3 µg/ml and the other two peptides at a concentration of 1 µg/l and measuring the reactivity towards MAb POX anti-P.malariae, gave the highest values with MAP[(NAAG)₆]₈. Approximately 50% of these values are obtained with (NAAG)₆NA-BSA and only 25% with (NAAG)₆-NA. With African sera the best results are also obtained with the synthetic peptide MAP[(NAAG)₆]₈.

### SPECIFICITY OF THE PEPTIPE MAP[(NAAG)₆]₈

### Reactivity with sera

Of the 88 Italian sera assayed, none gave an O.D. higher than 0.330. Of the 40 African sera, 4 gave O.D. values exceeding 1.200 and 2 exceeding 0.500.

34 Italian sera giving high absorbance on plates on which the peptide MAP[(NAAG)₆]₈ was adsorbed were assayed in parallel on pl ates on which the compound MAP[(NAAG)₆]₈ and (NANP)₄₀ were adsorbed. Slightly higher values were obtained with MAP[(NAAG)₆]₈ (X = 0.229) than with (NANP)₄₀ (X = 0.215).

### Reactivity with MAb anti P.malariae

The compound MAP[(NAAG)₆]₈ is recognized by monoclonal antisporozoite antibodies of P.malariae and does not react with MAb 2A10 directed against P. falciparum sporozoite.

The compound MAP[(NAAG)₆]₈ is recognized by antisporozoite MAb antibodies of P.malariae both in solution and when coupled to a solid phase.

Competitive experiments show that the antibodies present in a positive African serum recognize the same epitope as recognized by the antisporozoite MAb of P.malariae.

### EXAMPLE 3

### Immunization with MAP[(NAAG)₆]₈

### A) Production of antibodies

The ability of the compound MAP[(NAAG)₆]₈ to stimulate an in vivo antibody response is determined using mice of BALB/c (H-2^{d}), C57BL/6 (H-2^{b}), C3H/He (H-2^{k}) (Geneva University, switzerland), B10.G, B10.M, B10.S and B10.RIII (71NS) (supplied by Olac 1976 Ltd, Bicester U.K.) and C57BL/6nu/nu (GL Bomholtgard Ltd.,Ry, Denmark) types of about 8-12 weeks old of both sexes. Groups of 5 or 10 mice are treated intramuscularly at the base of the tail with an apyrogenic saline solution (50 µl) either alone or containing 50 µl of the peptides (NAAG)₆NA, Lys₇ [(K)₇-F-OH] (controls) and MAP[(NAAG)₆]₈, emulsified 1:1 in complete Freund's adjuvant (FCA). After 3 and 5 weeks the animals are again inoculated at the base of the tail with the same concentrations of peptides but in incomplete Freund's adjuvant (FIA).

Seven days after the last immunization, blood samples are taken from the retro-orbital plexus of the mice and the sera assayed by an ELISA to determine specific antibodies.

The suitably diluted sera samples are introduced into wells of a microplate on which MAP[(NAAG)₆]₈ (2 µg/ml) or the control peptide Lys₇ (10 µg/ml) have been adsorbed and are reacted overnight at 4°C. The antibodies linked to said antigens are detected after an incubation period of 3 hours at 37°C with the conjugate peroxidase-antimouse calf IgG antibodies (Zymed Laboratories Inc., San Francisco, CA) in the presence of the specific substrate (orthophenylenediamine of Fluka AG, Buschs, Switzerland: 0.4 mg/ml in 0.1 M citrate buffer pH 5.0 containing 0.01% of hydrogen peroxide). The enzymatic reaction is blocked after 20 minutes by adding 50 µl of 2.5 N H₂SO₄. The results are read after about 1 hour as optical density at 492 nm using a Titertek Multiskan reader (Flow Laboratories, McLean, VA). Serum samples giving OD values of less than 0.2 at a 1:100 dilution were considered negative.

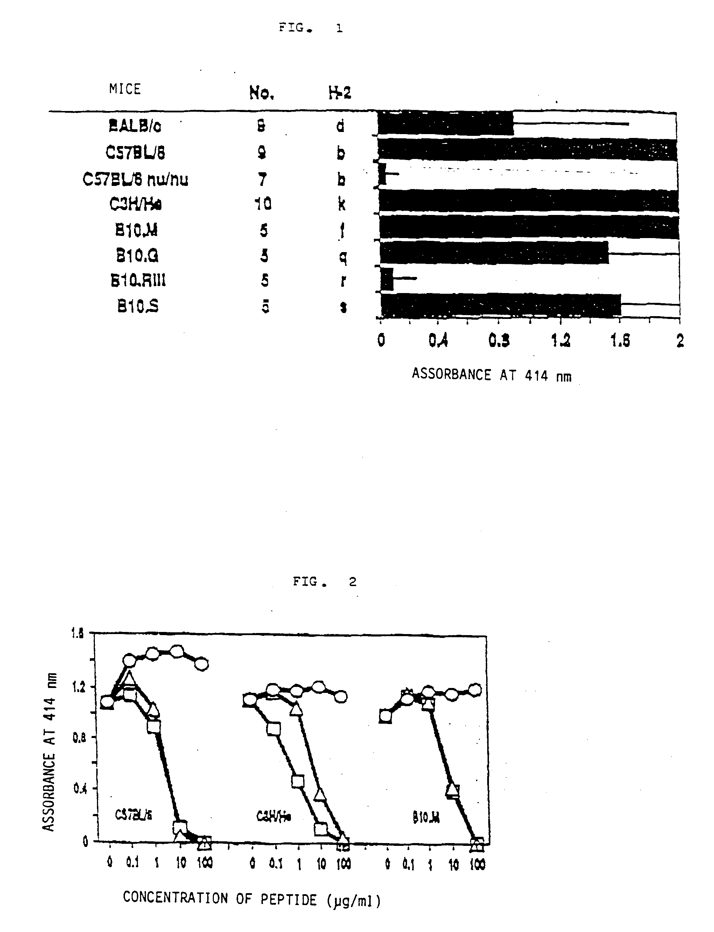

None of the mice innoculated with (NAAG)₆-NA induced antibody formation after three immunizations in complete Freund's adjuvant. As can be seen from Figure 1, anti-MAP[(NAAG)₆]₈ antibodies are determined in all serum samples with the exception of those from the B10.RIII (H-2^{r}) mice. The highest antibody values are encountered in the sera of B10.M (H-2^{F}), C57BL/6 (H-2^{b}) and C3H/He (H-2^{k}) mice; the lowest antibody levels are encountered in serum samples taken from BALB/c (H-2^{d}), B10.G (H-2^{q}) and B10.S (H-2^{s}) mice. No anti-MAP[(NAAG)₆]₈ antibody response is encountered in serum taken from mice C57BL/6nu/nu without thymus. No linkage of antibodies present in serum samples taken from mice immunized with the control peptide Lys₇ is determinable by the ELISA.

### B) Analysis of the specificity of anti-MAP[(NAAG)₆]₈ antibodies

The object of the experiment is to determine whether the antibodies induced by immunizing mice with the peptide MAP[(NAAG)₆]₈ are specific for the (NAAG)₆ epitope but not for the MAP polylysine core.

Serum samples taken from mice one week after their third immunization with 50 µg of MAP[(NAAG)₆]₈ in complete Freund's adjuvant are incubated at 37°C for 1.5 hours with different MAP[(NAAG)₆]₈, (NAAG)₆ or Lys₇ concentrations and then assayed by ELISA using plates on which the peptide MAP[(NAAG)₆]₈ is adsorbed (2 µg/ml). As shown in Figure 2 the ability of the antibodies present in the serum samples taken from C57BL/6, C3H/He and B10.M mice to bind to the peptide MAP[(NAAG)₆]₈ is completely inhibited both by the the same peptide and by the peptide (NAAG)₆-NA without the polylysine core, but never by the peptide Lys₇ without the NAAG component.

These results clearly show that the antibodies which develop after immunization with the peptide MAP[(NAAG)₆]₈ specifically recognize the repeating peptide (NAAG) of P.malariae but not the polylysine core contained in it.

### EXAMPLE 4

### Study of the carrier effect of the peptide MAP[(NAAG)₆]₈ on the production of (anti-NANP)₄₀ antibodies

The compound MAP[(NAAG)₆]₈ is conjugated with the peptide (NANP)₄₀ obtained as described in patent application EP-209,643, using 1-ethyl-3-(3-dimethylaminopropyl)-carbodiimide HCl (Sigma Chemical Co. St. Louis, MO) (Erlanger B.F., Methods Enzymol., 70: 85, 1980).

Groups of 5 BALB/c and C3H/He mice [non responders to the peptide (NANP)₄₀] and C57BL/6 mice [responders to the peptide (NANP)_{40]} are treated intramuscularly at the base of the tail with an apyrogenic saline solution (50 µl) either alone or containing 50 µl of the peptides (NANP)₄₀ or MAP[(NAAG)₆]₈-(NANP)₄₀, emulsified 1:1 in complete Freund's adjuvant (FCA). After 3 weeks the animals are again inoculated operating as just described. Seven days after the last immunization blood samples are taken from the retro-orbital plexus of the mice and the sera assayed by an ELISA as described in Example 3 using microplates on which the peptide (NANP)₄₀ has been adsorbed (1 µg/ml).

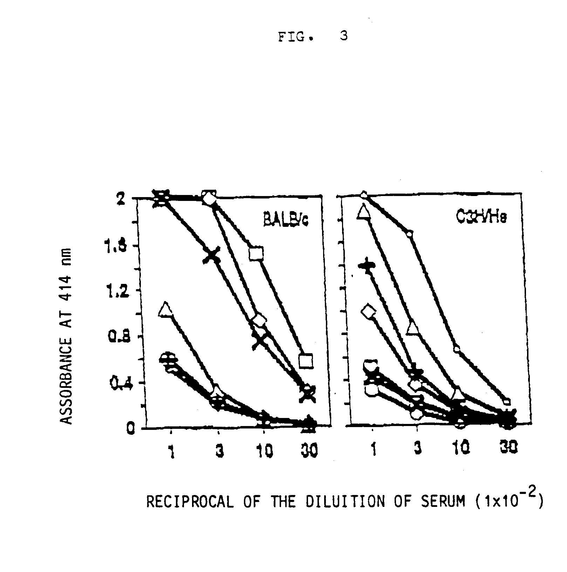

As can be seen from Figure 3 high anti-(NANP)₄₀ values are observable in all the mice examined. These results indicate that the peptide MAP[(NAAG)₆]₈ acts as carrier molecule in the production of anti-(NANP)₄₀ antibodies.

## Claims (Claims for the following Contracting State(s): AT, BE, CH, DE, DK, FR, GB, LI, LU, NL, SE)

1. An immunogenic compound having the general formula: 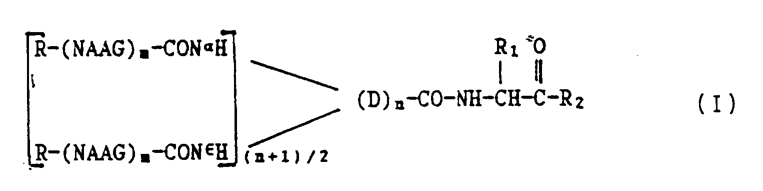 wherein:
n is an odd integer from 1 to 15
m is an integer from 3 to 40
N is L-Asp;
A is L-Ala;
G is L-Gly;
D is L-lysine, or a branched poly-(L-lysine) having a number, n, of L-lysine-amino-acid residues of an α and ε amide linkage;
R₁ is the side-chain of an amino-acid residue selected from L-Asp, L-His, L-Cys, L-Gln, L-Thr, L-Ala, L-Leu, L-Met, L-Phe, L-Glu, L-Arg, L-Tyr, L-Asn, L-Ser, L-Gly, L-Val, L-Ileu, L-Pro, and L-Trp;
R₂ is either -OH or -NH₂,
R is either hydrogen or an acyl,
and the pharmaceutically acceptable acid-addition or basic-addition salts thereof.

2. The immunogenic compound according to claim 1, wherein n is an odd integer from 3 to 7

3. The immunogenic compound according to Claim 1, wherein m is an integer from 6 to 15.

4. The immunogenic compound according to Claim 1, wherein R₁ is the side-chain of an amino-acid-residue other than N, A and G.

5. An immunogenic compound comprising one or more haptens having the general formula: 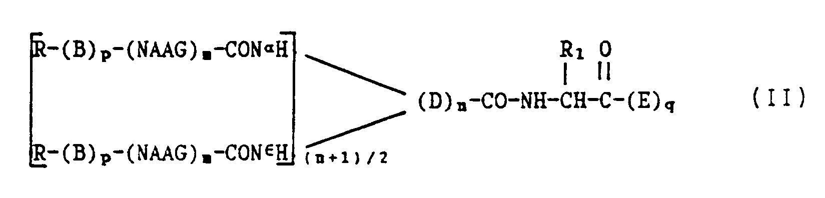 wherein:
n is an odd integer from 1 to 15;
m is an integer from 3 to 40;
N is L-Asp;
A is L-Ala;
G is L-Gly;
D is L-lysine, or a branched poly-(L-lysine) having a number, n, of L-lysine-amino-acid residues of α an and ε amide linkage;
R₁ is the side-chain of an amino-acid residue selected from L-Asp, L-His, L-Cys, L-Gln, L-Thr, L-Ala, L-Leu, L-Met, L-Phe, L-Glu, L-Arg, L-Tyr, L-Asn, L-Ser, L-Gly, L-Val, L-Ileu, L-Pro, and L-Trp;
R₂ is either -OH or -NH₂,
R is either hydrogen or an acyl,
B and E, equal to, or different from one another, are haptens selected from the B-epitopes of the CSP of Plasmodium falciparum, (NANP)ᵣ, and of Plasmodium vivax, (DRAD/AGQPAG)ᵣ, wherein r is an integer from 3 to 40;
p and q are 0 or 1 , with the proviso that they are never 0 simultaneously,
and the pharmaceutically acceptable acid-addition or basic-addition salts thereof.

6. The immunogenic compound according to Claim 5, wherein r is an integer from 5 to 16

7. Use of an immunogenic compound as defined in Claim 1, as an antigen in immunoenzymatic assays (EIA) directed to determining Plasmodium malariae antisporozoite antibodies in a sample of human blood, human blood serum, or human blood spot.

8. Use according to Claim 7, wherein the immunoenzymatic assay is an ELISA assay.

9. Use according to Claims 1, 7 and 8, wherein the ELISA assay is carried out by coating a solid support with the immunogenic compound (I), the concentration of said immunogenic compound in an adsorption buffer being of from 0,25 µg/ml to 2 µg/ml, the solid support thus obtained being then washed and dried.

10. Use according to Claim 9, wherein the adsorption buffer is Na₂CO₃.

11. Use according to Claim 9, wherein the concentration of the immunogenic compound (I) in the adsorption buffer is of from 0,5 µg/ml to 1,5 µg/ml.

12. Use according to Claims 9 and 11, wherein the concentration of the immunogenic compound (I) in the adsorption buffer is of from 0,8 µg/ml to 1,2 µg/ml.

13. A Plasmodium malariae antisporozoite vaccine composition inducing in a vertebrate host a genetically unrestricted protective immunity against infections caused by Plasmodium malariae, characterized in that it contains an immunogenically effective amount of a compound (I) as defined in Claim 1, or pharmaceutically acceptable acid-addition or basic-addition salts thereof.

14. A Plasmodium falciparum antisporozoite vaccine composition inducing in a vertebrate host a genetically unrestricted protective immunity against infections caused by Plasmodium falciparum, characterized in that it contains an immunogenically effective amount of a compound (II) as defined in Claim 5, wherein the hapten B and/or E is (NANP)ᵣ wherein r is an integer from 3 to 40, or pharmaceutically acceptable acid-addition or basic-addition salts thereof.

15. A Plasmodium vivaxantisporozoite vaccine composition inducing in a vertebrate host a genetically unrestricted protective immunity against infections caused by Plasmodium vivax, characterized in that it contains an immunogenically effective amount of a compound (II) as defined in Claim 5, wherein the hapten B and/or E is (DRAD/AGQPAG)ᵣ, wherein r is an integer from 3 to 40, or pharmaceutically acceptable acid-addition or basic-addition salts thereof.

## Claims (Claims for the following Contracting State(s): ES, GR)

1. Process for preparing the immunogenic compound having the general formula: 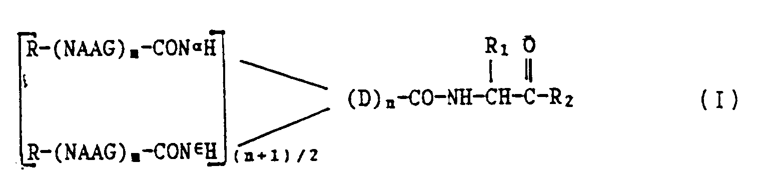 wherein:
n is an odd integer from 1 to 15
m is an integer from 3 to 40
N is L-Asp;
A is L-Ala;
G is L-Gly;
D is L-lysine, or a branched poly-(L-lysine) having a number, n, of L-lysine-amino-acid residues of an α and ε amide linkage;
R₁ is the side-chain of an amino-acid residue selected from L-Asp, L-His, L-Cys, L-Gln, L-Thr, L-Ala, L-Leu, L-Met, L-Phe, L-Glu, L-Arg, L-Tyr, L-Asn, L-Ser, L-Gly, L-Val, L-Ileu, L-Pro, and L-Trp;
R₂ is either -OH or -NH₂,
R is either hydrogen or an acyl,
characterized by comprising the steps of:
a) condensing the first amino-acid-residue protected at the α-amino group and optionally protected at the reactive side functions onto an insoluble substrate by esterifying the handle of the solid substrate with the activated terminal carboxylic group of the amino acid residue;
b) removing the α amino protecting group from the condensed amino-acid-residue;
c) condensing the L-lysine (K) amino-acid-residue protected at the α and ε amino groups by acylating the deprotected amino group of the amino-acid-residue bound to the insoluble solid substrate with the activated terminal carboxyl group of L-Lysine;
d) removing the α and ε amino protecting groups from the L-lysine amino acid residue;
e) condensing the L-lysine amino acid residues which are necessary to obtain the required polylysine core (D) by acylating the deprotected α and ε amino groups of the L-lysine residue and/or the residues bound to the support with the activated terminal carboxyl groups of the L-lysine residues;
f) removing the α and ε amino protecting groups from the L-lysine amino acid residues and condensing the next amino-acid-residues by acylating the deprotected and amino groups of the L-lysine amino-acid-residue and/or residues bound to the solid support with the activated terminal carboxyl group of the amino acis residues of the growing peptide chain with the activated terminal carboxyls until the compound (I) is completed, and
g) cleaving by acid hydrolysis the compound (I) thus obtained from its insoluble substrate, the compound (I) being purified by dialysis and/or chromatography.

2. Process according to Claim 1, characterized in that n is an odd integer from 3 to 7.

3. Process according to Claim 1, characterized in that m is an integer from 6 to 15.

4. Process according to Claim 1, characterized in that R1 is the side chain of an amino acid residue other than N, A and G.

5. Process according to Claim 1, characterized in that the insoluble solid substrate is a functionalized polyacrylamide resin.

6. Process according to Claim 1, characterized in that the protective group for the α-amino group is selected from:
benzyloxycarbonyl
triphenylmethyl
tert.amyloxycarbonyl
2-nitrophenylsulphonyl
(Fmoc, fluorenylmethyloxycarbonyl)
(Boc, tert.butyloxycarbonyl.

7. Process according to Claim 6, characterizd in that the protective group is fluorenylmethyloxycarbonyl (Fmoc) or tert.butyloxycarbonyl (Boc).

8. Process according to Claim 1, characterized in that the protective group for the reactive functional groups present in the amino acid side is selected:
for the carboxyl and hydroxyl of tyrosine, from the tert.butyl group and the tert.amyl group;
for the amino group of L-lysine, from the Boc group, the trifluoroacetyl group, the ortho-bromobenzoxycarbonyl (o-Brz) group and the benzyloxycarbonyl group;
for the guanidine group of arginine, from the trityl group and the N^{G}-4-methoxy-2,3,6 trimethyl-benzenesulphonyl (Mtr) group;
for histidine, from the triphenylmethyl group and the Boc group, and
is the tert.butylester (OBu^{t}) for the aspartic and glutamic acids.

9. Process according to Claim 1, characterized in that the terminal carboxyl group of the first amino acid residue is activated by reacting it with a phenol derivative to obtain the active ester at the terminal carboxyl.

10. Process according to Claim 9, characterized in that the phenol derivative is selected from pentafluorophenol, trichlorophenol, pentachlorophenol and p.nitrophenol.

11. Process according to Claim 1, characterized in that the esterification reaction of step (a) is carried out in the polar solvent dimethylformamide in the presence of 4-dimethylaminopyridine and N-methylmorpholine at the ambient temperature and for a time of 15 min.

12. Process according to Claim 1, characterized in that the acylation reaction of step (c) is carried out in an inert organic solvent selected from the chlorinated aliphatic hydrocarbons, the aliphatic ketones, and the alkyl esters, in the presence of 1-hydroxybenzotriazole as the catalyst, at a temperature of from -10°C to +40°C.

13. Process according to Claim 12, characterized in that the reaction temperature is of from 20°C to 25°C.

14. Process for preparing an immunogenic compound comprising one or more haptens, and having the general formula: 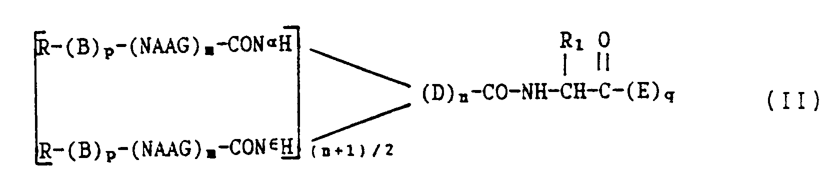 wherein:
n is an odd integer from 1 to 15;
m is an integer from 3 to 40;
N is L-Asp;
A is L-Ala;
G is L-Gly;
D is L-lysine, or a branched poly-(L-lysine) having a number, n, of L-lysine-amino-acid residues of an α and ε amide linkage;
R₁ is the side-chain of an amino-acid residue selected from L-Asp, L-His, L-Cys, L-Gln, L-Thr, L-Ala, L-Leu, L-Met, L-Phe, L-Glu, L-Arg, L-Tyr, L-Asn, L-Ser, L-Gly, L-Val, L-Ileu, L-Pro, and L-Trp;
R₂ is either -OH or -NH₂,
R is either hydrogen or an acyl,
B and E, equal to, or different from one another, are haptens selected from the B-epitopes of the CSP of Plasmodium falciparum, (NANP)ᵣ, and of Plasmodium vivax, (DRAD/AGQPAG)ᵣ, wherein r is an integer of from 3 to 40:
p and q are 0 or 1, with the proviso that they are never 0 simultaneously,
characterized in that it comprises the step of conjugating a compound of formula (I) as defined in Claim 1, wherein R₂ is -OH and R₁ is -H, with a hapten selected from the B-epitopes of the CSP of Plasmodium falciparum, (NANP)ᵣ, and of Plasmodium vivax, (DRAD/AGQPAG)ᵣ, wherein r is an integer of from 3 to 40.

15. Process according to Claim 14, characterized in that r is an integer from 5 to 16.

16. Process according to Claim 14, characterized in that the conjugation step is carried out either by homogeneous phase condensation in an inert organic solvent, or using a cross-linking agent of the glutaraldehyde type.

17. Process according to Claim 14, characterized in that the hapten is a polypeptide hapten and q is 0, the compound (II) being prepared by conjugating the protected amino acids by acylation at the (NAAG)ₘ until completing the hapten polypeptide sequence.

## Patentansprüche (Patentansprüche für folgende(n) Vertragsstaat(en): AT, BE, CH, DE, DK, FR, GB, LI, LU, NL, SE)

1. Immunogene Verbindung mit der allgemeinen Formel: 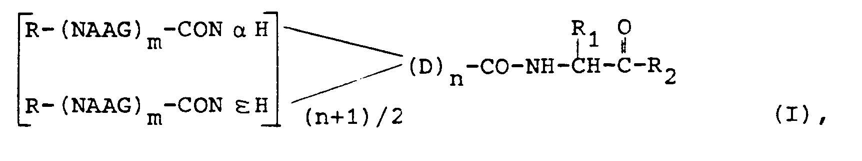 worin:
n eine ungerade ganze Zahl von 1 bis 15 ist;
m eine ganze Zahl von 3 bis 40 ist;
N für L-Asp steht;
A für L-Ala steht;
G für L-Gly steht;
D für L-Lysin oder ein verzweigtes Poly-(L-Lysin) steht, welches eine Anzahl n von L-Lysin-Aminosäureresten mit einer α- und ε-Amidbindung aufweist;
R₁ für die Seitenkette eines Aminosäurerestes steht, welcher aus L-Asp, L-His, L-Cys, L-Gln, L-Thr, L-Ala, L-Leu, L-Met, L-Phe, L-Glu, L-Arg, L-Tyr, L-Asn, L-Ser, L-Gly, L-Val, L-Ileu, L-Pro und L-Trp ausgewählt ist;
R₂ für entweder -OH oder -NH₂ steht; und
R für entweder Wasserstoff oder eine Acylgruppe steht; und die pharmazeutisch annehmbaren Säure- oder Basenadditionssalze hievon.

2. Immunogene Verbindung nach Anspruch 1, worin n eine ungerade ganze Zahl von 3 bis 7 darstellt.

3. Immunogene Verbindung nach Anspruch 1, worin m eine ganze Zahl von 6 bis 15 ist.

4. Immunogene Verbindung nach Anspruch 1, worin R₁ für die Seitenkette eines anderen Aminosäurerestes als N, A und G steht.

5. Immunogene Verbindung, welche ein oder mehrere Haptene enthält, und welche die allgemeine Formel: 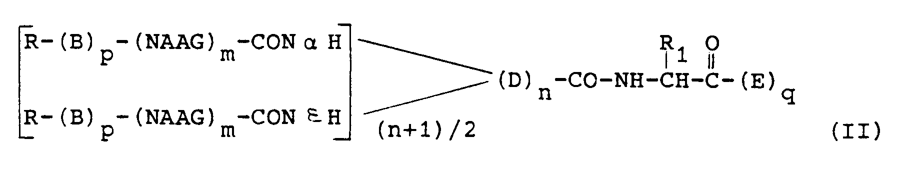 aufweist, worin:
n eine ungerade ganze Zahl von 1 bis 15 ist;
m eine ganze Zahl von 3 bis 40 ist;
N für L-Asp steht;
A für L-Ala steht;
G für L-Gly steht;
D für L-Lysin oder ein verzweigtes Poly-(L-Lysin) steht, welches eine Anzahl n von L-Lysin-Aminosäureresten mit einer α- und ε-Amidbindung aufweist;
R₁ für die Seitenkette eines Aminosäurerestes steht, welcher aus L-Asp, L-His, L-Cys, L-Gln, L-Thr, L-Ala, L-Leu, L-Met, L-Phe, L-Glu, L-Arg, L-Tyr, L-Asn, L-Ser, L-Gly, L-Val, L-Ileu, L-Pro und L-Trp ausgewählt ist;
R₂ für entweder -OH oder -NH₂ steht;
R für entweder Wasserstoff oder eine Acylgruppe steht;
B und E, welche gleich oder voneinander verschieden sind, Haptene sind, welche aus den B-Epitopen des CSP von Plasmodium falciparum, (NANP)ᵣ, und von Plasmodium vivax, (DRAD/AGQPAG)ᵣ, worin r eine ganze Zahl von 3 bis 40 darstellt, ausgewählt sind; und
p und q für 0 oder 1 stehen, mit der Maßgabe, daß sie niemals beide zugleich für Null stehen;
und die pharmazeutisch annehmbaren Säure- oder Basenadditionssalze hievon.

6. Immunogene Verbindung nach Anspruch 5, worin r eine ganze Zahl von 5 bis 16 darstellt.

7. Verwendung einer wie im Anspruch 1 definierten, immunogenen Verbindung als Antigen in Enzym-Immuntests (EIA), welche auf die Bestimmung von Antikörpern gegen die Sporozoiten von Plasmodium malariae in einer Probe von Humanblut, -blutserum oder in einem Humanbluttupfer gerichtet sind.

8. Verwendung nach Anspruch 7, wobei der Enzym-Immuntest ein ELISA-Test ist.

9. Verwendung nach den Ansprüchen 1, 7 und 8, wobei der ELISA-Test durchgeführt wird, indem man einen festen Träger mit der immunogenen Verbindung (I) überzieht, wobei die Konzentration dieser immunogenen Verbindung in einem Adsorptionspuffer 0,25 µg/ml bis 2 µg/ml beträgt, und indem man den so erhaltenen, festen Träger dann wäscht und trocknet.

10. Verwendung nach Anspruch 9, wobei der Adsorptionspuffer Na₂CO₃ ist.

11. Verwendung nach Anspruch 9, wobei die Konzentration der immunogenen Verbindung (I) in dem Adsorptionspuffer 0,5 µg/ml bis 1,5 µg/ml beträgt.

12. Verwendung nach den Ansprüchen 9 und 11, wobei die Konzentration der immunogenen Verbindung (I) in dem Adsorptionspuffer 0,8 µg/ml bis 1,2 µg/ml beträgt.

13. Vakzinezusammensetzung gegen die Sporozoiten von Plasmodium malariae, welche in einem Wirbeltier-Wirtsorganismus eine genetisch unbeschränkte, protektive Immunität gegen durch Plasmodium malariae hervorgerufene Infektionen induziert, dadurch gekennzeichnet, daß sie eine immunogen wirksame Menge einer wie im Anspruch 1 definierten Verbindung (I) oder von pharmazeutisch annehmbaren Säure- oder Basenadditionssalzen hievon enthält.

14. Vakzinezusammensetzung gegen die Sporozoiten von Plasmodium falciparum, welche in einem Wirbeltier-Wirtsorganismus eine genetisch unbeschränkte, protektive Immunität gegen durch Plasmodium falciparum hervorgerufene Infektionen induziert, dadurch gekennzeichnet, daß sie eine immunogen wirksame Menge einer wie im Anspruch 5 definierten Verbindung (II), worin das Hapten B und/oder E (NANP)ᵣ ist, worin r eine ganze Zahl von 3 bis 40 darstellt, oder von pharmazeutisch annehmbaren Säure- oder Basenadditionssalzen hievon enthält.

15. Vakzinezusammensetzung gegen die Sporozoiten von Plasmodium vivax, welche in einem Wirbeltier-Wirtsorganismus eine genetisch unbeschränkte, protektive Immunität gegen durch Plasmodium vivax hervorgerufene Infektionen induziert, dadurch gekennzeichnet, daß sie eine immunogen wirksame Menge einer wie im Anspruch 5 definierten Verbindung (II), worin das Hapten B und/oder E (DRAD/AGQPAG)ᵣ ist, worin r eine ganze Zahl von 3 bis 40 darstellt, oder von pharmazeutisch annehmbaren Säure- oder Basenadditionssalzen hievon enthält.

## Patentansprüche (Patentansprüche für folgende(n) Vertragsstaat(en): ES, GR)

1. Verfahren zur Herstellung einer immunogenen Verbindung mit der allgemeinen Formel: 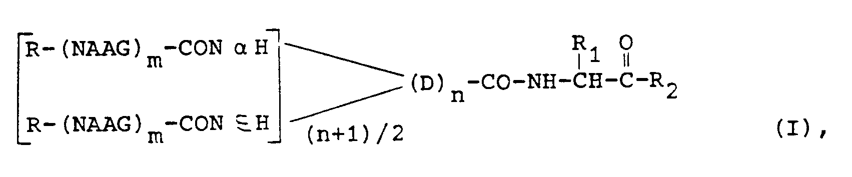 worin:
n eine ungerade ganze Zahl von 1 bis 15 ist;
m eine ganze Zahl von 3 bis 40 ist;
N für L-Asp steht;
A für L-Ala steht;
G für L-Gly steht;
D für L-Lysin oder ein verzweigtes Poly-(L-Lysin) steht, welches eine Anzahl n von L-Lysin-Aminosäureresten mit einer α- und ε-Amidbindung aufweist;
R₁ für die Seitenkette eines Aminosäurerestes steht, welcher aus L-Asp, L-His, L-Cys, L-Gln, L-Thr, L-Ala, L-Leu, L-Met, L-Phe, L-Glu, L-Arg, L-Tyr, L-Asn, L-Ser, L-Gly, L-Val, L-Ileu, L-Pro und L-Trp ausgewählt ist;
R₂ für entweder -OH oder -NH₂ steht; und
R für entweder Wasserstoff oder eine Acylgruppe steht;
dadurch gekennzeichnet, daß es die folgenden Stufen umfaßt:
a) Kondensieren des ersten Aminosäurerestes, welcher an der α-Aminogruppe geschützt ist, und welcher gegebenenfalls an den reaktiven Seitenkettenfunktionen geschützt ist, an ein unlösliches Substrat durch Verestern des Verankerungsteiles des festen Substrates mit der aktivierten, endständigen Carboxylgruppe des Aminosäurerestes;
b) Entfernen der α-Amino-Schutzgruppe von dem kondensierten Aminosäurerest;
c) Kondensieren des an den α- und ε-Aminogruppen geschützten L-Lysin-(K)-Aminosäurerestes durch Acylieren der von der Schutzgruppe befreiten Aminogruppe des an das unlösliche, feste Substrat gebundenen Aminosäurerestes mit der aktivierten, endständigen Carboxylgruppe des L-Lysins;
d) Entfernen der α- und ε-Amino-Schutzgruppen aus dem L-Lysin-Aminosäurerest;
e) Kondensieren der L-Lysin-Aminosäurereste, welche zur Erzielung des erforderlichen Polylysinkernes (D) erforderlich sind, durch Acylieren der von ihren Schutzgruppen befreiten α- und ε-Aminogruppen des L-Lysinrestes und/oder der an den Träger gebundenen Reste mit den aktivierten, endständigen Carboxylgruppen der L-Lysinreste;
f) Entfernen der α- und ε-Amino-Schutzgruppen von den L-Lysin-Aminosäureresten und Kondensieren der nächsten Aminosäurereste durch Acylieren der von ihren Schutzgruppen befreiten Aminogruppen der L-Lysin-Aminosäurereste und/oder der an den festen Träger gebundenen Reste mit der aktivierten, endständigen Carboxylgruppe der Aminosäurenreste der wachsenden Peptidkette mit den aktivierten, endständigen Carboxylgruppen so lange, bis die Verbindung (I) fertiggestellt worden ist, und
g) Abspalten der so erhaltenen Verbindung (I) von ihrem unlöslichen Substrat, durch Säurehydrolyse, wobei die Verbindung (I) durch Dialyse und/oder Chromatographie gereinigt wird.

2. Verfahren nach Anspruch 1, dadurch gekennzeichnet, daß n eine ungerade ganze Zahl von 3 bis 7 darstellt.

3. Verfahren nach Anspruch 1, dadurch gekennzeichnet, daß m eine ganze Zahl von 6 bis 15 ist.

4. Verfahren nach Anspruch 1, dadurch gekennzeichnet, daß R₁ für die Seitenkette eines anderen Aminosäurerestes als N, A und G steht.

5. Verfahren nach Anspruch 1, dadurch gekennzeichnet, daß das unlösliche feste Substrat ein funktionalisiertes Polyacrylamidharz ist.

6. Verfahren nach Anspruch 1, dadurch gekennzeichnet, daß die Schutzgruppe für die α-Aminogruppe aus:
Benzyloxycarbonyl
Triphenylmethyl
tert.-Amyloxycarbonyl
2-Nitrophenylsulfonyl
(Fmoc, Fluorenylmethyloxycarbonyl)
(Boc, tert.-Butyloxycarbonyl)
ausgewählt wird.

7. Verfahren nach Anspruch 6, dadurch gekennzeichnet, daß die Schutzgruppe Fluorenylmethyloxycarbonyl (Fmoc) oder tert.-Butyloxycarbonyl (Boc) ist.

8. Verfahren nach Anspruch 1, dadurch gekennzeichnet, daß die Schutzgruppe für die in der Aminosäureseitenkette vorhandenen, reaktiven funktionellen Gruppen ausgewählt ist:
für die Carboxyl- und Hydroxylgruppe des Tyrosins aus der tert.-Butylgruppe und der tert.-Amylgruppe;
für die Aminogruppe des L-Lysins aus der Boc-Gruppe, der Trifluoracetylgruppe, der ortho-Brombenzoxycarbonyl-(o-Brz)-Gruppe und der Benzyloxycarbonylgruppe;
für die Guanidingruppe des Arginins aus der Tritylgruppe und der N^{G}-4-Methoxy-2,3,6-trimethyl-benzolsulfonyl-(Mtr)-Gruppe;
für Histidin aus der Triphenylmethylgruppe und der Boc-Gruppe; und
für Asparaginsäure und Glutaminsäure der tert.-Butylester (OBu^{t}) ist.

9. Verfahren nach Anspruch 1, dadurch gekennzeichnet, daß die endständige Carboxylgruppe des ersten Aminosäurerestes aktiviert wird, indem man sie mit einem Phenolderivat umsetzt, um an der endständigen Carboxylgruppe den aktiven Ester zu erhalten.

10. Verfahren nach Anspruch 9, dadurch gekennzeichnet, daß das Phenolderivat aus Pentafluorphenol, Trichlorphenol, Pentachlorphenol und p-Nitrophenol ausgewählt wird.

11. Verfahren nach Anspruch 1, dadurch gekennzeichnet, daß die Veresterungsreaktion der Stufe (a) in dem polaren Lösungsmittel Dimethylformamid in Gegenwart von 4-Dimethylaminopyridin und N-Methylmorpholin bei Umgebungstemperatur und während einer Zeitspanne von 15 min durchgeführt wird.

12. Verfahren nach Anspruch 1, dadurch gekennzeichnet, daß die Acylierungsreaktion der Stufe (c) in einem inerten organischen Lösungsmittel, welches aus chlorierten aliphatischen Kohlenwasserstoffen, aliphatischen Ketonen und Alkylestern ausgewählt ist, und in Gegenwart von 1-Hydroxybenzotriazol als Katalysator bei einer Temperatur von -10°C bis +40°C durchgeführt wird.

13. Verfahren nach Anspruch 12, dadurch gekennzeichnet, daß die Reaktionstemperatur 20°C bis 25°C beträgt.

14. Verfahren zur Herstellung einer immunogenen Verbindung, welche ein oder mehrere Haptene enthält, und welche die allgemeine Formel: 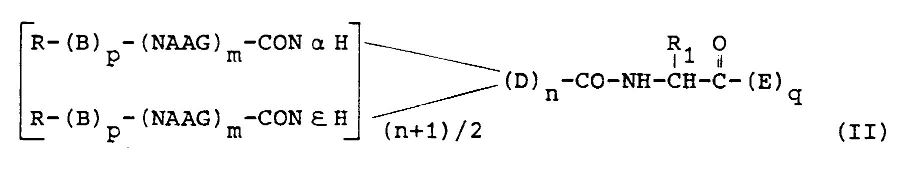 aufweist, worin:
n eine ungerade ganze Zahl von 1 bis 15 ist;
m eine ganze Zahl von 3 bis 40 ist;
N für L-Asp steht;
A für L-Ala steht;
G für L-Gly steht;
D für L-Lysin oder ein verzweigtes Poly-(L-Lysin) steht, welches eine Anzahl n von L-Lysin-Aminosäureresten mit einer α- und ε-Amidbindung aufweist;
R₁ für die Seitenkette eines Aminosäurerestes steht, welcher aus L-Asp, L-His, L-Cys, L-Gln, L-Thr, L-Ala, L-Leu, L-Met, L-Phe, L-Glu, L-Arg, L-Tyr, L-Asn, L-Ser, L-Gly, L-Val, L-Ileu, L-Pro und L-Trp ausgewählt ist;
R₂ für entweder -OH oder -NH₂ steht;
R für entweder Wasserstoff oder eine Acylgruppe steht;
B und E, welche gleich oder voneinander verschieden sind, Haptene sind, welche aus den B-Epitopen des CSP von Plasmodium falciparum, (NANP)ᵣ, und von Plasmodium vivax, (DRAD/AGQPAG)ᵣ, worin r eine ganze Zahl von 3 bis 40 darstellt, ausgewählt sind; und
p und q für 0 oder 1 stehen, mit der Maßgabe, daß sie niemals beide zugleich für Null stehen;
dadurch gekennzeichnet, daß dieses Verfahren die Stufen des Konjugierens einer wie im Anspruch 1 definierten Verbindung der Formel (I), worin R₂ für -OH steht und R₁ für -H steht, mit einem Hapten, welches aus den B-Epitopen des CSP von Plasmodium falciparum, (NANP)ᵣ , und von Plasmodium vivax, (DRAD/AGQPAG)ᵣ, worin r eine ganze Zahl von 3 bis 40 ist, ausgewählt ist, umfaßt.

15. Verfahren nach Anspruch 14, dadurch gekennzeichnet, daß r eine ganze Zahl von 5 bis 16 darstellt.

16. Verfahren nach Anspruch 14, dadurch gekennzeichnet, daß die Stufe der Konjugatbildung entweder durch Kondensation in homogener Phase in einem inerten organischen Lösungsmittel oder unter Verwendung eines Vernetzungsmittels vom Glutaraldehydtypus durchgeführt wird.

17. Verfahren nach Anspruch 14, dadurch gekennzeichnet, daß das Hapten ein Polypeptid-Hapten ist, und daß q für Null steht, und daß die Verbindung (II) hergestellt wird, indem man die geschützten Aminosäuren durch Acylierung an dem (NAAG)ₘ so lange konjugiert, bis die Hapten-Polypeptid-Sequenz fertiggestellt ist.

## Revendications (Revendications pour l'(les) Etat(s) contractant(s) suivant(s): AT, BE, CH, DE, DK, FR, GB, LI, LU, NL, SE)

1. Composé immunogène de formule générale : 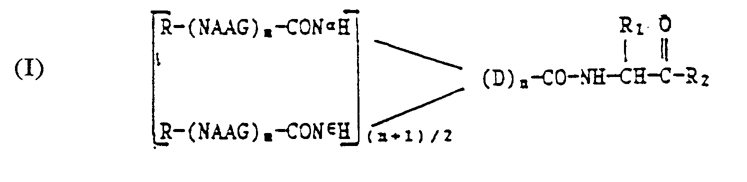 dans laquelle
n représente un nombre entier impair valant de 1 à 15,
m représente un nombre entier valant de 3 à 40,
N représente L-Asp;
A représente L-Ala,
G représente L-Gly,
D représente la L-lysine, ou une poly(L-lysine) ramifiée comportant un nombre n de résidus d'acide aminé L-lysine, à liaisons peptidiques en α et en ε,
R₁ représente la chaîne latérale d'un résidu d'acide aminé choisi parmi L-Asp, L-His, L-Cys, L-Gln, L-Thr, L-Ala, L-Leu, L-Met, L-Phe, L-Glu, L-Arg, L-Tyr, L-Asn, L-Ser, L-Gly, L-Val, L-Ile, L-Pro et L-Trp,
R₂ représente -OH ou -NH₂, et
R représente un atome d'hydrogène ou un groupe acyle,
ainsi que les sels d'addition d'acide et les sels d'addition de base d'un tel composé.

2. Composé immunogène conforme à la revendication 1, dans lequel n représente un nombre entier impair valant de 3 à 7.

3. Composé immunogène conforme à la revendication 1, dans lequel m représente un nombre entier valant de 6 à 15.

4. Composé immunogène conforme à la revendication 1, dans lequel R₁ représente la chaîne latérale d'un résidu d'acide aminé autre que N, A ou G.

5. Composé immunogène comprenant un ou plusieurs haptènes, de formule générale : 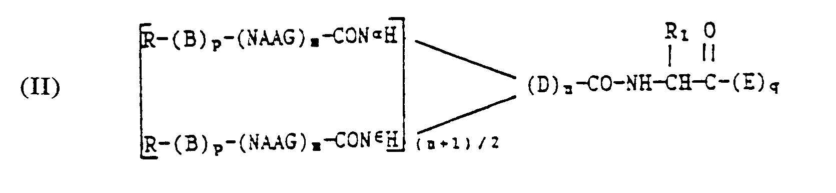 dans laquelle
n représente un nombre entier impair valant de 1 à 15,
m représente un nombre entier valant de 3 à 40,
N représente L-Asp,
A représente L-Ala,
G représente L-Gly,
D représente la L-lysine, ou une poly(L-lysine) ramifiée comportant un nombre n de résidus d'acide aminé L-lysine, à liaisons peptidiques en α et en ε,
R₁ représente la chaîne latérale d'un résidu d'acide antiné choisi parmi L-Asp, L-His, L-Cys, L-Gln, L-Thr, L-Ala, L-Leu, L-Met, L-Phe, L-Glu, L-Arg, L-Tyr, L-Asn, L-Ser, L-Gly, L-Val, L-Ile, L-Pro et L-Trp,
R₂ représente -OH ou -NH₂,
R représente un atome d'hydrogène ou un groupe acyle,
B et E, identiques ou différents l'un de l'autre, représentent des haptènes choisis parmi les épitopes B de la CSP de Plasmodium falciparum (NANP)ᵣ ou de Plasmodium vivax (DRAD/AGQPAG)ᵣ, r représentant un nombre entier valant de 3 à 40, et
p et q valent chacun 0 ou 1, à condition qu'ils ne vaillent pas 0 simultanément,
ainsi que les sels d'addition d'acide et les sels d'addition de base d'un tel composé.

6. Composé immunogène conforme à la revendication 5, dans lequel r représente un nombre entier valant de 5 à 16.

7. Emploi d'un composé immunogène conforme à la revendication 1, en tant qu'antigène dans des tests immunoenzymatiques (EIA) effectués dans le but de détecter des anticorps dirigés contre les sporozoïtes de Plasmodium malariae dans un échantillon de sang humain ou de sérum sanguin humain, ou dans une tache de sang humain.

8. Emploi conforme à la revendication 7, dans lequel le test immunoenzymatique est un test ELISA.

9. Emploi conforme aux revendications 1, 7 et 8, dans lequel on effectue le test ELISA en revêtant un support solide avec un composé immunogène (I), la concentration de ce composé immunogène dans un tampon d'adsorption valant de 0,25 µg/ml à 2 µg/ml, et le support solide ainsi obtenu étant ensuite lavé et séché.

10. Emploi conforme à la revendication 9, dans lequel le tampon d'adsorption est à base de Na₂CO₃.

11. Emploi conforme à la revendication 9, dans lequel la concentration du composé immunogène (I) dans le tampon d'adsorption vaut de 0,5 µg/ml à 1,5 µg/ml.

12. Emploi conforme aux revendications 9 et 11, dans lequel la concentration du composé immunogène (I) dans le tampon d'adsorption vaut de 0,8 µg/ml à 1,2 µg/ml.

13. Composition de vaccin antisporozoïtique contre Plasmodium malariae, faisant apparaître, chez un hôte vertébré, une immunité protectrice, non génétiquement restreinte, contre les infections provoquées par Plasmodium malariae, caractérisée en ce qu'elle contient une quantité, efficace pour l'immunisation, d'un composé (I) conforme à la revendication 1, ou de l'un des sels d'addition d'acide ou de base d'un tel composé, acceptables en pharmacie.

14. Composition de vaccin antisporozoïtique contre Plasmodium falciparum, faisant apparaître, chez un hôte vertébré, une immunité protectrice, non génétiquement restreinte, contre les infections provoquées par Plasmodium falciparum, caractérisée en ce qu'elle contient une quantité, efficace pour l'immunisation, d'un composé (II) conforme à la revendication 5, dans lequel le haptène B et/ou E est (NANP)ᵣ où r représente un nombre entier valant de 3 à 40, ou de l'un des sels d'addition d'acide ou de base d'un tel composé, acceptables en pharmacie.

15. Composition de vaccin antisporozoïtique contre Plasmodium vivax, faisant apparaître, chez un hôte vertébré, une immunité protectrice, non génétiquement restreinte, contre les infections provoquées par Plasmodium vivax, caractérisée en ce qu'elle contient une quantité, efficace pour l'immunisation, d'un composé (II) conforme à la revendication 5, dans lequel le haptène B et/ou E est (DRAD/AGQPAG)ᵣ où r représente un nombre entier valant de 3 à 40, ou de l'un des sels d'addition d'acide ou de base d'un tel composé, acceptables en pharmacie.

## Revendications (Revendications pour l'(les) Etat(s) contractant(s) suivant(s): ES, GR)

1. Procédé de préparation d'un composé immunogène de formule générale : 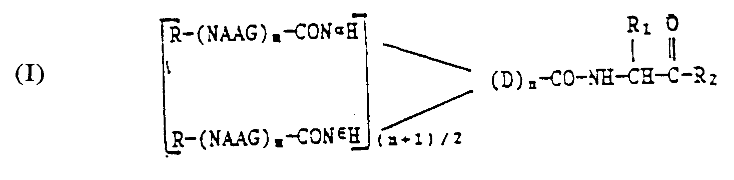 dans laquelle
n représente un nombre entier impair valant de 1 à 15,
m représente un nombre entier valant de 3 à 40,
N représente L-Asp,
A représente L-Ala,
G représente L-Gly,
D représente la L-lysine, ou une poly(L-lysine) ramifiée comportant un nombre n de résidus d'acide aminé L-lysine, à liaisons peptidiques en α et en ε,
R₁ représente la chaîne latérale d'un résidu d'acide aminé choisi parmi L-Asp, L-His, L-Cys, L-Gln, L-Thr, L-Ala, L-Leu, L-Met, L-Phe, L-Glu, L-Arg, L-Tyr, L-Asn, L-Ser, L-Gly, L-Val, L-Ile, L-Pro et L-Trp,
R₂ représente -OH ou -NH₂, et
R représente un atome d'hydrogène ou un groupe acyle,
procédé caractérisé en ce qu'il comporte les étapes consistant à :
a) condenser le premier résidu d'acide aminé, dont le groupe α-amino est protégé, ainsi qu'éventuellement les groupes fonctionnels latéraux réactifs, sur un substrat insoluble, par estérification de la poignée d'accrochage du substrat solide avec le groupe carboxy terminal activé du résidu d'acide aminé ;
b) enlever le groupe α-amino-protecteur du résidu condensé d'acide aminé;
c) condenser le résidu de L-lysine (K), dont les groupes amino en α et ε sont protégés, par acylation du groupe amino déprotégé du résidu d'acide aminé lié au substrat solide insoluble avec le groupe carboxy terminal activé de la L-lysine;
d) enlever les groupes protégeant les groupes amino en α et ε du résidu de L-lysine;
e) condenser les résidus de L-lysine nécessaires pour obtenir le noyau de polylysine (D) requis, par acylation des groupes amino en α et ε déprotégés des résidus de L-lysine et/ou des résidus liés au support avec les groupes carboxy terminaux activés de résidus de L-lysine;
f) enlever les groupes protégeant les groupes amino en α et ε des résidus de L-lysine et condenser les résidus suivants d'acide aminé, par acylation des groupes amino en α et ε déprotégés des résidus de L-lysine et/ou des résidus liés au support solide avec les groupes carboxy terminaux activés des résidus d'acide aminé de la chaîne peptidique en croissance, avec les groupes carboxy terminaux activés, jusqu'a' l'achèvement du composé (I); et
g) séparer le composé (I) de son substrat insoluble, par hydrolyse acide, le composé (I) étant purifié par dialyse et/ou chromatographie.

2. Procédé conforme à la revendication 1, caractérisé en ce que n représente un nombre entier impair valant de 3 à 7.

3. Procédé conforme à la revendication 1, caractérisé en ce que m représente un nombre entier valant de 6 à 15.

4. Procédé conforme à la revendication 1, caractérisé en ce que R₁ représente la chaîne latérale d'un résidu d'acide aminé autre que N, A ou G.

5. Procédé conforme à la revendication 1, caractérisé en ce que le substrat solide insoluble est une résine de polyacrylamide fonctionnalisée.

6. Procédé conforme à la revendication 1, caractérisé en ce que le groupe protecteur du groupe α-amino est choisi parmi les groupes :
benzyloxycarbonyle
triphénylméthyle
tertioamyloxycarbonyle
2-nitrophénylsulfonyle
fluorénylméthyloxycarbonyle (Fmoc)
tertiobutyloxycarbonyle (Boc)

7. Procédé conforme à la revendication 6, caractérisé en ce que le groupe protecteur est un groupe fluorénylméthyloxycarbonyle (Fmoc) ou tertiobutyloxycarbonyle (Boc).

8. Procédé conforme à la revendication 1, caractérisé en ce que les groupes protecteurs des groupes fonctionnels réactifs présents dans les chaînes latérales des acides aminés sont choisis :
- pour le groupe carboxy et le groupe hydroxy de la tyrosine, parmi le groupe tertiobutyle et le groupe tertioamyle;
- pour le groupe amino de la L-lysine, parmi le groupe Boc, le groupe trifluoroacétyle, le groupe ortho-bromobenzoxycarbonyle (o-Brz) et le groupe benzyloxycarbonyle ;
- pour le groupe guanidino de l'arginine, parmi le groupe trityle et le groupe N^{G}-4-méthoxy-2,3,6-triméthyl-benzènesulfonyle (Mtr) ;
- pour l'histidine, parmi le groupe triphénylméthyle et le groupe Boc ; et en ce qu'il s'agit du groupe tertiobutyloxy (OBu^{t}) pour les acides glutamique et aspartique.

9. Procédé conforme à la revendication 1, caractérisé en ce que l'on active le groupe carboxy terminal du premier résidu d'acide aminé en le faisant réagir avec un dérivé du phénol pour obtenir un ester actif au niveau du groupe carboxy terminal.

10. Procédé conforme à la revendication 9, caractérisé en ce que le dérivé de phénol est choisi parmi le pentafluorophénol, un trichlorophénol, le pentachlorophénol et le p-nitrophénol.

11. Procédé conforme à la revendication 1, caractérisé en ce que l'on effectue la réaction d'estérification de l'étape (a) dans un solvant polaire, du diméthylformamide, et en présence de 4-diméthylaminopyridine et de N-méthylmorpholine, à la température ambiante et pendant une durée de 15 minutes.

12. Procédé conforme à la revendication 1, caractérisé en ce que l'on effectue la réaction d'acylation de l'étape (c) dans un solvant organique inerte choisi parmi les hydrocarbures aliphatiques chlorés, les cétones aliphatiques et les esters d'alkyle, en présence de 1-hydroxybenzotriazole utilisé en tant que catalyseur, et à une température valant de -10°C à +40°C.

13. Procédé conforme à la revendication 12, caractérisé en ce que la température de réaction vaut de 20°C à 25°C.

14. Procédé de préparation d'un composé immunogène comprenant un ou plusieurs haptènes, de formule générale : 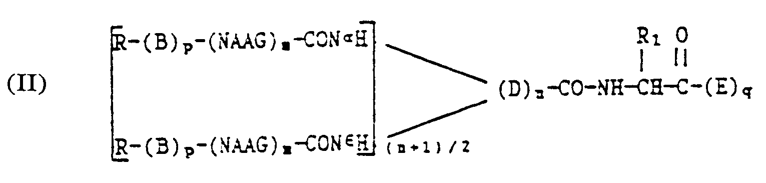 dans laquelle
n représente un nombre entier impair valant de 1 à 15,
m représente un nombre entier valant de 3 à 40,
N représente L-Asp,
A représente L-Ala,
G représente L-Gly,
D représente la L-lysine, ou une poly(L-lysine) ramifiée comportant un nombre n de résidus d'acide aminé L-lysine, à liaisons peptidiques en α et en ε,
R₁ représente la chaîne latérale d'un résidu d'acide aminé choisi parmi L-Asp, L-His, L-Cys, L-Gln, L-Thr, L-Ala, L-Leu, L-Met, L-Phe, L-Glu, L-Arg, L-Tyr, L-Asn, L-Ser, L-Gly, L-Val, L-Ile, L-Pro et L-Trp,
R₂ représente -OH ou -NH₂,
R représente un atome d'hydrogène ou un groupe acyle,
B et E, identiques ou différents l'un de l'autre, représentent des haptènes choisis parmi les épitopes B de la CSP de Plasmodium falciparum (NANP)ᵣ ou de Plasmodium vivax (DRAD/AGQPAG)ᵣ, r représentant un nombre entier valant de 3 à 40, et
p et q valent chacun 0 ou 1, à condition qu'ils ne vaillent pas 0 simultanément,
procédé caractérisé en ce qu'il comporte l'étape consistant à conjuguer un composé de formule (I), défini dans la revendication 1, dans lequel R₂ représente -OH et R₁ représente -H, avec un haptène choisi parmi les épitopes B de la CSP de Plasmodium falciparum (NANP)ᵣ ou de Plasmodium vivax(DRAD/AGQPAG)ᵣ, r représentant un nombre entier valant de 3 à 40.

15. Procédé conforme à la revendication 14, caractérisé en ce que r représente un nombre entier valant de 5 à 16.

16. Procédé conforme à la revendication 14, caractérisé en ce que l'on effectue l'étape de conjugaison par condensation en phase homogène dans un solvant organique inerte, ou en utilisant un agent de liaison de type glutaraldéhyde.

17. Procédé conforme à la revendication 14, caractérisé en ce que le haptène est un haptène polypeptidique et q vaut 0, le composé (II) étant préparé par conjugaison des acides aminés protégés, par acylation au niveau de (NAAG)ₘ, jusqu'à l'achèvement de la séquence polypeptidique du haptène.
